# EUROPEAN PATENT SPECIFICATION

(11) **EP 0 497 919 B1**
(45) Date of publication and mention of the grant of the patent: **11.12.1996**
(21) Application number: 91900624.7
(22) Date of filing: 23.10.1990
(51) Int. Cl.: A61N 1/30

(54) **Iontophoresis device using a rate-controlling membrane**
Vorrichtung für Iontophorese unter Verwendung einer Durchfluss-kontrollierenden Membran
Dispositif d'iontophorése utilisant und membrane regulatrice du débit

(30) Priority: 23.10.1989 US 427069
(43) Date of publication of application: 12.08.1992
(73) Proprietor: THERATECH, INC. (a Delaware Corporation), Salt Lake City, Utah 84108 (US)
(72) Inventor: VENKATESHWARAN, Srinivasan, Salt Lake City, UT 84102 (US); CHENG, Daniel, C., H., Salt Lake City, UT 84121 (US)
(74) Representative: Goldin, Douglas Michael
(86) International application number: PCT/US90/06121
(87) International publication number: WO 91/05582

(56) References cited:
- FR-A- 2 562 800
- US-A- 4 722 726
- US-A- 4 731 049
- US-A- 4 781 924
- US-A- 4 820 770
- US-A- 4 837 027

## Description

This invention is in the field of transdermal drug delivery. More particularly it relates to the iontophoretic administration of drugs through the skin.

Iontophoresis is a well-known manner of administering through the skin. See, for instance, U.S. Patents Nos. 4,744,787, 4,722,726, 4,731,049 and 4,752,285.

The use of rate-controlling membranes in the passive (non-iontophoretic) administration of drugs transdermally is described in U.S. Patents Nos. 3,598,122, 3,797,444 and 3,966,934.

The use of skin permeation enhancers to increase the permeability of skin to drugs is well known. See, for instance, U.S. Patents Nos. 4,006,218, 4,568,343, 4,746,515, 3,989,816, 4,316,893, 4,764,379 and EP Publications 272,987, 261,429 and 043738.

US-A-4,722,726 describes an iontophoretic device having a microporous membrane. When present, the membrane serves to separate physically a drug-containing reservoir from the skin. Under the influence of an electric field, the drug migrates through the microporous membrane. There is no disclosure or suggestion that the membrane is electrically sensitive.

US-A-4,781,924 describes a transdermal device that comprises a non-hydrated drug-containing reservoir and a membrane that is impermeable to the non-hydrated but permeable to the hydrated form the of the drug. Moisture from the skin migrates up through the membrane to the reservoir where it hydrates the drug. Once hydrated, the drug diffuses from the reservoir through the membrane to the skin.

One aspect of the invention is a device for administering a drug iontophoretically through a predetermined area of skin in a therapeutically effective regimen comprising:
(a) a first reservoir containing the drug in a form susceptible to iontophoretic administration in communication with said area of skin;
(b) a skin permeation enhancing agent contained within said reservoir;
(c) a first electrode in communication with said first reservoir;
(d) a second reservoir containing an electrolyte in communication with the skin at a location separated from said area of skin;
(e) a second electrode in electrical communication with the second reservoir;
(f) means for creating an electric voltage difference between the first and second electrodes; and
(g) an electrically sensitive membrane interposed between said first reservoir and the area of skin, said membrane being substantially impermeable to the drug when the voltage difference is absent and permeable to the drug when the voltage difference is present.

Optionally the second reservoir contains a drug in a form susceptible to iontophoretic administration.

Another embodiment of the invention is a device for administering a drug iontophoretically through a predetermined area of skin in a therapeutically effective regimen comprising:
(a) a first reservoir containing the drug in a form susceptible to iontophoretic administration in communication with said area of skin;
(b) a skin permeation enhancing agent contained within a second reservoir that is also in communication with said area of skin;
(c) a first electrode in communication with said first reservoir;
(d) a third reservoir containing an electrolyte in communication with the skin at a location separated from said area of skin;
(e) a second electrode in electrical communication with the third reservoir;
(f) means for creating an electric voltage difference between the first and second electrodes; and
(g) an electrically sensitive membrane interposed between said first reservoir and the area of skin, said membrane being substantially impermeable to the drug when the voltage difference is absent and is permeable to the drug when the voltage difference is present.

This device may further comprise a second electrically sensitive membrane interposed between the third reservoir and the skin at a location separated from said first area of skin, said membrane being substantially impermeable to the drug when the voltage difference is absent and permeable to the drug when the voltage difference is present.

Alternatively this device may further comprise a skin permeation enhancing agent contained within a fourth reservoir that is also in communication with the skin at said location separated from said first area of skin; and a second electrically sensitive membrane interposed between the third reservoir and the skin at a location separated from said first area of skin, said membrane being substantially impermeable to the drug when the voltage difference is absent and permeable to the drug when the voltage difference is present.

The device of the invention may be used in a method of administering a drug iontophoretically through a predetermined area of the skin of a patient comprising: placing a first reservoir in the form of a solution or a gel of the drug in communication with said area of skin, said first reservoir having a first electrode in electrical communication therewith; placing a second reservoir containing an electrolyte in communication with the skin at a location separated from said area, said second reservoir having a second electrode in electrical communication therewith; and creating a voltage difference between the first and second electrodes, whereby a skin permeation enhancer is applied to the skin to make the skin substantially permeable to the drug and an electrically sensitive membrane is interposed between the reservoir and the area of the skin, the membrane being substantially impermeable to the drug in the absence of the voltage difference and permeable to the drug in the presence of the voltage difference.

### Brief Description of the Drawings

Figure 1 is an exploded view of an embodiment of the invention device.

Figure 2 is a schematic showing the placement of the device on the skin.

### Modes For Carrying Out The Invention

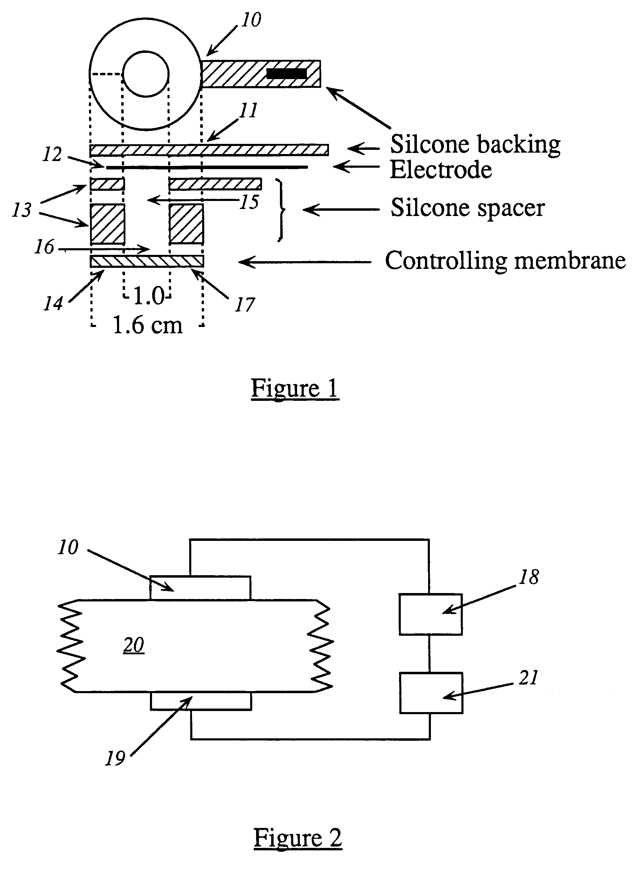

Figure 1 depicts an iontophoresis device, generally designated 10. Device 10 consists of an impermeable backing sheet 11 (such as Dow Corning silastic sheeting), an electrode 12, an impermeable spacer element 13 which can be made of the same material as backing 11, and a rate-controlling membrane 14. In one embodiment of this device that was used to delivery the model drug TEAB (tetraethylammonium bromide; (CH₃CH₂)₄-N⁺·Br⁻), a membrane made of 25 micrometer thick laminate of cellophane coated with polyvinylchloride or saran to render the surface hydrophobic was used. The cylindrical space 15 within the spacer is filled with a formulation of the drug in ionic form (solution or gel), designated 16 in Figure 1.

In use, device 10 is placed on the skin with the basal surface 17 of the membrane in contact with the skin. If desired, the device can be affixed to the skin by means of an adhesive overlay (not shown) or a layer or peripheral ring of drug-permeable adhesive on the basal surface of the membrane (not shown). Electrode 12 is then connected to a battery or other D.C. power source 18 (Figure 2). The polarity of electrode 12 will depend upon the charge of the ion species of drug. The drug and electrode 12 should be of the same charge. A second electrode contained in an assembly 19 similar to 10 which contains an electrolyte, and optionally, drug and permeation enhancer is placed on the skin 20 at a location separated from electrode 12. When assembly 19 contains drug and permeation enhancer, a rate controlling membrane (not shown) is interposed between the reservoir of assembly 19 and the skin. The electrode in assembly 19 is connected to the opposite pole of the power source. The circuit preferably includes conventional switching, voltage regulation and timing means, schematically designated 21, to control the duration and magnitude of the current flowing through the circuit.

A central feature of this invention is the employment of membrane 14 as an element that monitors the rate at which the drug is administered to the patient. In order for this to happen two conditions must be met. First, the skin itself must not be a rate-controlling barrier to delivery of the drug from the device through to circulation (in other words, the skin is "substantially permeable" to the drug). To achieve this condition, a skin permeation enhancing agent is applied to the area of skin in contact with device 10 through which the current is driven into the skin. That application may be made prior to or concurrently with the drug administration. When administered concurrently, the enhancer is included as a component of the device. For instance, the enhancer may be contained in the reservoir with the drug or in a second reservoir layer that either overlies or underlies the membrane, depending upon whether the membrane is permeable to the enhancer. Normally, if the membrane is impermeable to the enhancer, the enhancer will be contained in a second, underlying reservoir layer. Alternatively in such instances, and depending upon the particular enhancer used, the drug ions may act as carriers to transport the enhancer through the membrane. Examples of permeation enhancers are given in the patents and patent publications mentioned above.

The second condition is that the membrane be electrically sensitive; that is, the membrane is substantially impermeable to the drug in the absence of the voltage difference across the electrodes and permeable when the difference is applied. Accordingly, the pattern of drug delivery is essentially controlled by the permeability of the membrane to the drug, which, in turn is controlled electrically. Thus, by manipulation of the switching/voltage regulation/timing means in the circuit, an infinite variety of temporal or preprogrammed dosing regimens may be achieved. Zero order (constant rate) release rate is achieved by keeping the current through the circuit constant and the transference number of the drug ion constant. In general, the rate of administration increases with increasing current.

Preferably, the electrodes are silver-silver chloride. Examples of other electrodes used in iontophoresis devices are described at col. 5 of U.S. Patent No. 4,744,787.

When silver-silver chloride electrodes are employed, the drug can be delivered from both devices (10 and 19) in an alternating regimen. In this case both devices would be identical except that one would contain a silver electrode and the other a silver chloride electrode. The drug would be present in both devices and the polarity would be alternated periodically to deliver the drug from one or the other device. The drug will be delivered from the device whose polarity is the same as the sign of the charge on the drug. Such a configuration (delivering drug from both devices in an alternating fashion) can prevent significant depletion of drug in the device, which would be more likely to occur if it were delivered from a single device. Also reversing polarity reverses the electrochemical reactions at the electrodes and regenerates the original electrode material (silver or silver chloride). This in turn helps maintain the pH of the drug reservoir in the two devices constant during iontophoresis. Better control of a drug depletion in the device and the pH of the drug reservoir can maintain the transference number of the drug ion constant for much longer periods, thus allowing zero order (constant) input of the drug for prolonged periods.

The backing and spacer element of device 10 may be made from drug impermeable materials such as those described in the patents cited above.

The conditions, e.g., voltage difference, current, pH at electrodes, used to administer drugs iontophoretically are described in the patents cited above.

While the invention has been described specifically in terms of an embodiment that is suited to input a drug iontophoretically, it will be appreciated that the device in a slightly modified form can be employed for noninvasive sampling of tissue fluids transdermally. In devices 10 and 19, the rate limiting membrane can be replaced by a microporous (non-rate limiting) membrane, and in conjunction with a skin permeation enhancer within the device or in a second reservoir that is in communication with the skin, these devices can be used to noninvasively sample tissue fluids. When an electric current is driven from one device to the other and the skin itself is not a rate controlling barrier, charged substances in the tissue fluid can be noninvasively sampled at the appropriate device (10 or 19) depending on the polarity. Uncharged species (e.g., glucose) in the tissue fluid can also be noninvasively sampled because they are transported during iontophoresis by the current induced convective solvent flow. Uncharged species in the tissue fluid can be sampled at either device (10 or 19) depending on the direction of the convection flow of tissue fluid solvent (water).

## Claims

1. A device (10, 19) for administering a drug iontophoretically through a predetermined area of skin in a therapeutically effective regimen comprising:
(a) a first reservoir (15) containing the drug in a form susceptible to iontophoretic administration in communication with said area of skin;
(b) a skin permeation enhancing agent contained within said reservoir;
(c) a first electrode (12) in communication with said first reservoir;
(d) a second reservoir containing an electrolyte in communication with the skin at a location separated from said area of skin;
(e) a second electrode in electrical communication with the second reservoir;
(f) means (18) for creating an electric voltage difference between the first and second electrodes; and
(g) an electrically sensitive membrane (14) interposed between said first reservoir and the area of skin, said membrane being substantially impermeable to the drug when the voltage difference is absent and permeable to the drug when the voltage difference is present.

2. The device of claim 1, wherein the second reservoir contains a drug in a form susceptible to iontophoretic administration.

3. A device for administering a drug iontophoretically through a predetermined area of skin in a therapeutically effective regimen comprising:
(a) a first reservoir containing the drug in a form susceptible to iontophoretic administration in communication with said area of skin;
(b) a skin permeation enhancing agent contained within a second reservoir that is also in communication with said area of skin;
(c) a first electrode in communication with said first reservoir;
(d) a third reservoir containing an electrolyte in communication with the skin at a location separated from said area of skin;
(e) a second electrode in electrical communication with the third reservoir;
(f) means for creating an electric voltage difference between the first and second electrodes; and
(g) an electrically sensitive membrane interposed between said first reservoir and the area of skin, said membrane being substantially impermeable to the drug when the voltage difference is absent and is permeable to the drug when the voltage difference is present.

4. The device of claim 3, wherein said third reservoir contains a drug in a form susceptible to iontophoretic administration and a skin permeation enhancing agent and said device further comprises:
(h) a second electrically sensitive membrane interposed between the third reservoir and the skin at a location separated from said first area of skin, said membrane being substantially impermeable to the drug when the voltage difference is absent and permeable to the drug when the voltage difference is present.

5. The device of claim 3, wherein the third reservoir contains a drug in a form susceptible to iontophoretic administration and said device further comprises:
(h) a skin permeation enhancing agent contained within a fourth reservoir that is also in communication with the skin at said location separated from said first area of skin; and
(i) a second electrically sensitive membrane interposed between the third reservoir and the skin at a location separated from said first area of skin, said membrane being substantially impermeable to the drug when the voltage difference is absent and permeable to the drug when the voltage difference is present.

## Patentansprüche

1. Vorrichtung (10, 19) zum Verabreichen eines Medikaments iontophoretisch durch einen vorbestimmten Hautbereich bei einer therapeutisch wirksamen Maßnahme, wobei die Vorrichtung folgendes umfaßt:
(a) einen ersten Behälter (15), der das Medikament in einer Form enthält, die die iontophoretische Verabreichung in Verbindung mit diesem Hautbereich zuläßt,
(b) ein in diesem Behälter enthaltenes Mittel, das die Durchdringung der Haut verbessert,
(c) eine erste Elektrode (12) in Verbindung mit dem ersten Behälter,
(d) einen zweiten Behälter, der einen Elektrolyten in Verbindung mit der Haut an einer von dem besagten Hautbereich entfernten Stelle enthält,
(e) eine zweite Elektrode in elektrischer Verbindung mit dem zweiten Behälter,
(f) Mittel (18) zum Schaffen einer elektrischen Spannungsdifferenz zwischen den ersten und zweiten Elektroden, und
(g) eine auf Elektrizität ansprechende Membran (14), die zwischen dem ersten Behälter und dem Hautbereich angeordnet ist, wobei die Membran für das Medikament im wesentlich undurchlässig ist, wenn keine Spannungsdifferenz vorhanden ist, und für das Medikament durchlässig ist, wenn die Spannungsdifferenz vorhanden ist.

2. Vorrichtung nach Anspruch 1, bei der der zweite Behälter ein Medikament in einer Form enthält, die für die iontophoretische Verabreichung empfänglich ist.

3. Vorrichtung zum Verabreichen eines Medikaments iontophoretisch durch einen vorbestimmten Hautbereich bei einer therapeutisch wirksamen Maßnahme, wobei die Vorrichtung folgendes umfaßt:
(a) einen ersten Behälter, der das Medikament in einer Form enthält, die die iontophoretische Verabreichung in Verbindung mit dem Hautbereich zuläßt,
(b) ein Mittel zur Verbesserung der Durchdringung der Haut, das in einem zweiten Behälter, der ebenfalls in Verbindung mit dem besagten Hautbereich steht, enthalten ist,
(c) eine erste Elektrode in Verbindung mit dem ersten Behälter,
(d) einen dritten Behälter, der einen Elektrolyten in Verbindung mit der Haut an einer von dem besagten Hautbereich entfernten Stelle enthält,
(e) eine zweite Elektrode in elektrischer Verbindung mit dem dritten Behälter,
(f) Mittel zum Schaffen einer elektrischen Spannungsdifferenz zwischen den ersten und zweiten Elektroden, und
(g) eine auf Elektrizität ansprechende Membran, die zwischen dem ersten Behälter und dem Hautbereich angeordnet ist, wobei die Membran für das Medikament im wesentlich undurchlässig ist, wenn keine Spannungsdifferenz vorhanden ist, und für das Medikament durchlässig ist, wenn die Spannungsdifferenz vorhanden ist.

4. Vorrichtung nach Anspruch 3, bei der der dritte Behälter ein Medikament in einer Form, die für die iontophoretische Verabreichung suszeptibel ist, und ein Mittel zur Verbesserung der Durchdringung der Haut enthält, und wobei die Vorrichtung desweiteren folgendes umfaßt:
(h) eine zweite auf Elektrizität ansprechende Membran, die zwischen dem dritten Behälter und der Haut an einer von dem ersten Hautbereich entfernten Stelle angeordnet ist, wobei die Membran für das Medikament im wesentlich undurchlässig ist, wenn keine Spannungsdifferenz vorhanden ist, und für das Medikament durchlässig ist, wenn die Spannungsdifferenz vorhanden ist.

5. Vorrichtung nach Anspruch 3, bei der der dritte Behälter ein Medikament in einer Form enthält, die die iontophoretische Verabreichung zuläßt, und die Vorrichtung desweiteren folgendes umfaßt:
(h) ein Mittel zur Verbesserung der Durchdringung der Haut, das in einem vierten Behälter enthalten ist, der ebenfalls mit der Haut an einer von dem ersten Hautbereich getrennten Stelle in Verbindung steht, und
(i) eine zweite auf Elektrizität ansprechende Membran, die zwischen dem dritten Behälter und der Haut an einer von dem ersten Hautbereich entfernten Stelle angeordnet ist, wobei die Membran für das Medikament im wesentlich undurchlässig ist, wenn keine Spannungsdifferenz vorhanden ist, und für das Medikament durchlässig ist, wenn die Spannungsdifferenz vorhanden ist.

## Revendications

1. Dispositif (10, 19) pour administrer un médicament par ionophorèse à travers une surface de peau prédéterminée dans le cadre d un régime à but thérapeutique, comprenant:
(a) un premier réservoir (15) contenant, le médicament sous une forme se prêtant à une administration par ionophorèse, en communication avec ladite surface de peau;
(b) un agent favorisant la pénétration percutanée, contenu dans ledit réservoir;
(c) une première électrode (12) en communication avec ledit premier réservoir;
(d) un second réservoir contenant un électrolyte, en communication avec la peau à un emplacement séparé de ladite surface de peau;
(e) une seconde électrode en communication électrique avec le second réservoir;
(f) un moyen (18) pour créer une différence de tension électrique entre les première et seconde électrodes; et
(g) une membrane (14) électrosensible intercalée entre ledit premier réservoir et la surface de peau, ladite membrane étant sensiblement imperméable au médicament en l'absence de la différence de tension et perméable au médicament en présence de la différence de tension.

2. Dispositif selon la revendication 1, dans lequel le second réservoir contient un médicament sous une forme se prêtant à une administration par ionophorèse.

3. Dispositif (10, 19) pour administrer un médicament par ionophorèse à travers une surface de peau prédéterminée dans le cadre d'un régime à but thérapeutique, comprenant:
(a) un premier réservoir contenant le médicament sous une forme se prêtant à une administration par ionophorèse, en communication avec ladite surface de peau;
(b) un agent favorisant la pénétration percutanée, contenu dans un second réservoir qui est lui aussi en communication avec ladite surface de peau;
(c) une première électrode en communication avec ledit premier réservoir;
(d) un troisième réservoir contenant un électrolyte, en communication avec la peau à un emplacement séparé de ladite surface de peau;
(e) une seconde électrode en communication électrique avec le troisième réservoir;
(f) un moyen (18) pour créer une différence de tension électrique entre les première et seconde électrodes; et
(g) une membrane électrosensible intercalée entre ledit premier réservoir et la surface de peau, ladite membrane étant sensiblement imperméable au médicament en l'absence de la différence de tension et perméable au médicament en présence de la différence de tension.

4. Dispositif selon la revendication 3, dans lequel ledit troisième réservoir contient un médicament sous une forme se prêtant à une administration par ionophorèse et un agent favorisant la pénétration percutanée, et ledit dispositif comprend en outre:
(h) une membrane électrosensible intercalée entre le troisième réservoir et la peau à un emplacement séparé de ladite première surface de peau, ladite membrane étant sensiblement imperméable au médicament en l'absence de la différence de tension et perméable au médicament en présence de la différence de tension.

5. Dispositif selon la revendication 3, dans lequel le troisième réservoir contient un médicament sous une forme se prêtant à une administration par ionophorèse et ledit dispositif comprend en outre :
(h) un agent favorisant la pénétration percutanée, contenu dans un quatrième réservoir qui est lui aussi en communication avec la peau audit emplacement séparé de ladite première surface de peau; et
(i) une seconde membrane électrosensible intercalée entre le troisième réservoir et la peau à un emplacement séparé de ladite première surface de peau, ladite membrane étant sensiblement imperméable au médicament en l'absence de la différence de tension et perméable au médicament en présence de la différence de tension.
